# FASCICULE DE BREVET EUROPEEN

(11) **EP 0 850 081 B1**
(45) Date de publication et mention de la délivrance du brevet: **08.03.2000**
(21) Numéro de dépôt: 96929378.6
(22) Date de dépôt: 29.08.1996
(51) Int. Cl.: A61M 15/00

(54) **APPAREIL D'INHALATION DESTINE A DISTRIBUER DES DOSES PRECISES ET REPRODUCTIBLES DE PRODUIT PULVERULENT**
INHALATIONSVORRICHTUNG ZUR GENAUEN UND REPRODUZIERBAREN, DATIERTEN ABGABE VON PULVERFÖRMIGEN ARZNEISTOFFEN
INHALING APPARATUS INTENDED TO DISPENSE PRECISE AND REPRODUCIBLE DOSES OF PULVERULENT PRODUCT

(30) Priorité: 04.09.1995 FR 9510354
(43) Date de publication de la demande: 01.07.1998
(73) Titulaire: VALOIS S.A., 27110 Le Neubourg (FR)
(72) Inventeur: BRUNA, Pascal, F-76000 Rouen (FR); FOURMENT, Olivier, F-75016 Paris (FR); STRADELLA, Guiseppe, I-16032 Camogli (IT)
(74) Mandataire: CAPRI SARL
(86) Numéro de dépôt international: FR9601328
(87) Numéro de publication internationale: WO9709083

(56) Documents cités:
- EP-A- 0 424 790
- EP-A- 0 573 128
- WO-A-92/04068
- WO-A-93/16748
- WO-A-93/18812

## Description

La présente invention concerne un appareil d'inhalation et plus particulièrement un distributeur de poudre sèche (DPI/BAI) actif, actionné par l'inhalation de l'utilisateur.

Par DPI actif, on entend un dispositif de distribution de poudre dans lequel la dose de poudre est distribuée au moyen d'un écoulement d'air créé indépendamment de l'inhalation de l'utilisateur. Ces dispositifs trouvent leur application principale dans la distribution de produits pulvérulents, en particulier de médicaments, qui sont destinés à être distribués à l'utilisateur de manière précisément dosée, à un moment précisément déterminé, ainsi qu'à un endroit précisément déterminé, en l'occurrence les poumons. Ceci est notamment le cas de certains médicaments destinés aux asthmatiques, où il est souhaitable qu'une quantité de produit la plus reproductible possible parvienne le plus profondément à l'intérieur des poumons du patient. Pour ce faire, il est essentiel que la dose de produit expulsée par l'appareil soit déterminée de manière précise et reproductible, que les doses de produit soient expulsées toujours dans les mêmes conditions, et que ces expulsions soient coordonnées avec l'inhalation du patient (BAI actif).

Un tel dispositif est décrit notamment dans le document WO 93/18812. Ce document divulgue un inhalateur comportant un réservoir de poudre et des moyens de dosage permettant, à chaque actionnement de l'inhalateur, de remplir une chambre de dosage avec une dose de produit, l'appareil comprenant en outre une source d'air comprimé déclenchable par l'inhalation et qui crée un courant d'air comprimé venant chasser la dose de poudre située dans la chambre de dosage.

Selon le type de poudre ou de médicament utilisé et selon l'utilisation pour laquelle l'appareil est destiné, le dispositif divulgué dans le document WO 93/18812 peut présenter certains inconvénients. Ainsi un conditionnement de l'ensemble de la poudre dans un seul réservoir implique qu'en cas de contamination et/ou d'humidification d'une partie de ladite poudre, l'ensemble du produit risque d'être contaminé et/ou humidifié. D'autre part, dans le cas de certaines poudres ayant des molécules fragiles, par exemple formées de longues chaînes, celles-ci risquent de casser pendant l'opération de dosage, ce qui peut altérer l'efficacité du produit. De plus, le réservoir peut comporter certains volumes morts et la poudre contenue dans ces volumes morts ne pourra pas être transférée dans la chambre de dosage par les moyens de dosage, ce qui représente un inconvénient lorsque le produit en question est très coûteux. En outre, la reproductibilité de la dose n'est pas parfaite.

Le document WO 93/16748 divulgue un appareil d'inhalation comportant un dispositif de transfert de doses de produit à partir d'un réservoir commun. Ce dispositif permet d'améliorer la reproductibilité de la dose, mais présente toujours les autres inconvénients précités.

Dans le document WO 94/08552, il est divulgué un appareil comportant un dispositif de pré-dosage sous forme de réservoirs individuels de la poudre, l'appareil comprenant en outre un moteur électrique déclenché par l'inhalation de l'utilisateur, ledit moteur électrique créant un courant d'air pour chasser une dose lorsque le patient inhale. Ce dispositif présente un inconvénient majeur en ce qu'il comporte des moyens électriques pour distribuer la dose, ce qui en augmente considérablement les coûts, le rend difficile à fabriquer, et le rend dépendant d'une source d'énergie électrique pour fonctionner.

La présente invention a pour but de fournir un appareil d'inhalation ne comportant pas les inconvénients précités et fonctionnant de manière optimale. Pour fonctionner de manière optimale, un tel appareil doit remplir simultanément chacune des conditions suivantes de manière optimale:
- la totalité de la poudre contenue dans l'appareil doit être protégée de toute contamination et/ou d'humidification extérieure,
- la totalité de la poudre contenue dans l'appareil doit être distribuée par dose très exactement reproductible,
- l'expulsion de la dose doit être coordonnée avec l'inhalation du patient,
- chaque dose de poudre doit pénétrer finement pulvérisée profondément dans les poumons de l'utilisateur.

La présente invention a aussi pour but de fournir un appareil d'inhalation capable de distribuer de manière précise et économique la totalité du produit contenu dans l'appareil, ce produit ne devant pas être altéré d'une quelconque façon.

La présente invention a donc pour objet un appareil d'inhalation destiné à distribuer des doses précises et reproductibles de produit pulvérulent comme défini dans la revendication 1.

Ainsi, c'est la combinaison des moyens techniques précités qui permet de résoudre le problème technique de l'invention : le dispositif de pré-dosage en réservoirs individuels permet d'éviter le risque de contamination et/ou d'humidification de la totalité du produit. En effet, même si la poudre contenue dans l'un des réservoirs est contaminée et/ou humidifiée, le restant de la poudre n'est pas affecté. De plus, le dispositif de pré-dosage garantit la parfaite reproductibilité de la dose, puisque chaque réservoir contient exactement une dose, celle-ci étant transférée en vue de son expulsion au moyen du dispositif de transfert. Ensuite, la source d'écoulement d'air permet de créer un courant d'air très exactement reproductible adapté à expulser les différentes doses de poudre dans les mêmes conditions. De même, cet écoulement d'air permet, si nécessaire, de désagglomérer la dose de poudre avant son expulsion et ainsi garantir qu'elle soit finement pulvérisée, ce qui favorise sa pénétration dans les poumons. Ceci est encore amplifié, du fait que l'expulsion de la dose est coordonnées avec l'inhalation de l'utilisateur.

Avantageusement ladite pompe est précontrainte et comporte un piston coulissant dans une chambre reliée audit canal d'expulsion, ledit piston étant sollicité par un ressort, ledit ressort étant comprimable manuellement et ledit piston étant retenu contre la force exercée par ledit ressort comprimé par un organe de blocage, ledit organe de blocage étant libéré par l'inhalation de l'utilisateur, de sorte que le ressort se détend en déplaçant ledit piston dans ladite chambre, créant ainsi un écoulement d'air dans ledit canal d'expulsion.

Avantageusement, ledit dispositif de pré-dosage comporte un support pourvu d'au moins un réservoir, chaque réservoir comportant une dose précise de produit, le dispositif de transfert comportant des moyens d'entraînement dudit support pour amener un desdits réservoirs dans le canal d'expulsion avant une inhalation de l'utilisateur.

D'autres avantages et caractéristiques de l'invention apparaîtront au cours de la description détaillée suivante donnée à titre d'exemple non limitatif au regard des dessins joints, sur lesquels :
- la figure 1 représente une vue schématique en coupe d'un appareil d'inhalation selon un mode de réalisation particulier de l'invention,
- la figure 2 représente une vue schématique en coupe d'un mode de réalisation particulier de la source d'écoulement d'air pour un appareil d'inhalation selon l'invention,
- les figures 3a et 3b représentent des vues schématiques en coupe d'un mode de réalisation particulier des dispositifs de pré-dosage et de transfert d'un appareil d'inhalation selon l'invention, respectivement en position de repos et d'actionnement du dispositif de transfert,
- la figure 3c représente une vue de dessus du dispositif de pré-dosage des figures 3a et 3b,
- la figure 3d représente une vue latérale d'une partie du dispositif de transfert des figures 3a et 3b, et
- les figures 4a, 4b et 4c sont des vues similaires à celles des figures 3a, 3b et 3c respectivement, représentant un autre mode de réalisation des dispositifs de pré-dosage et de transfert selon l'invention.

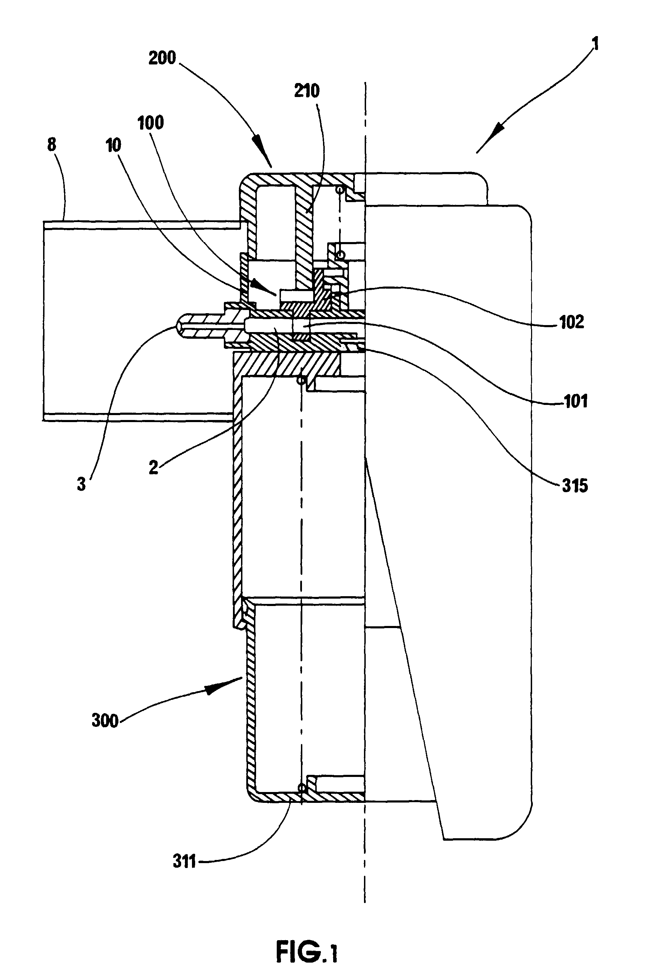

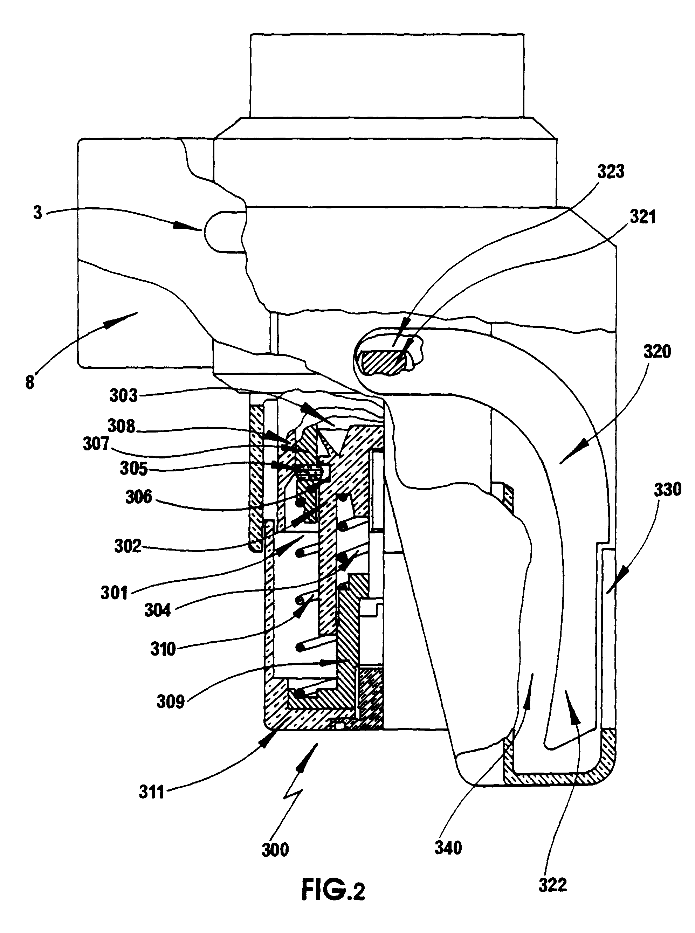

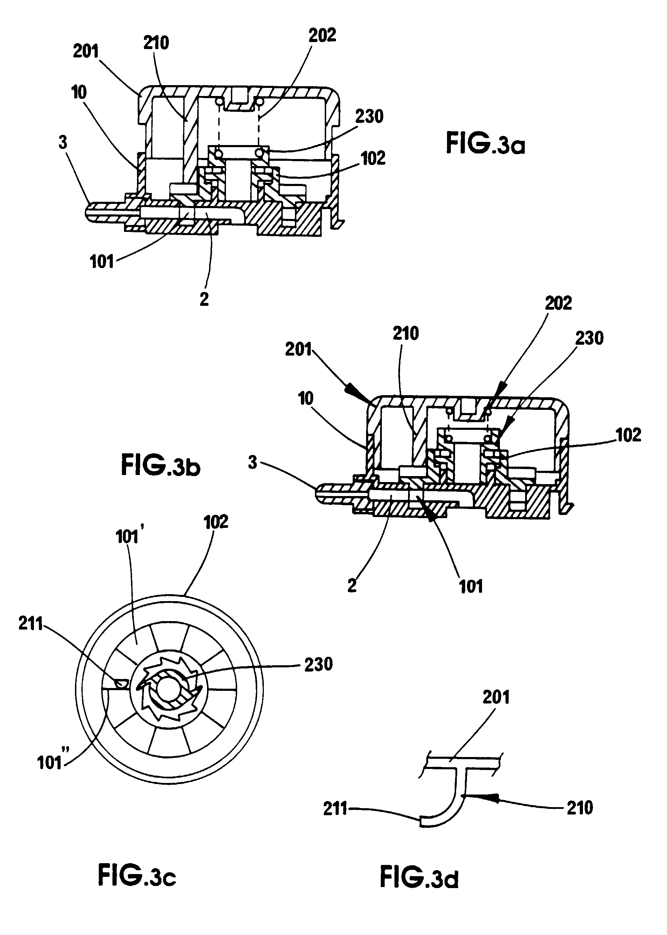

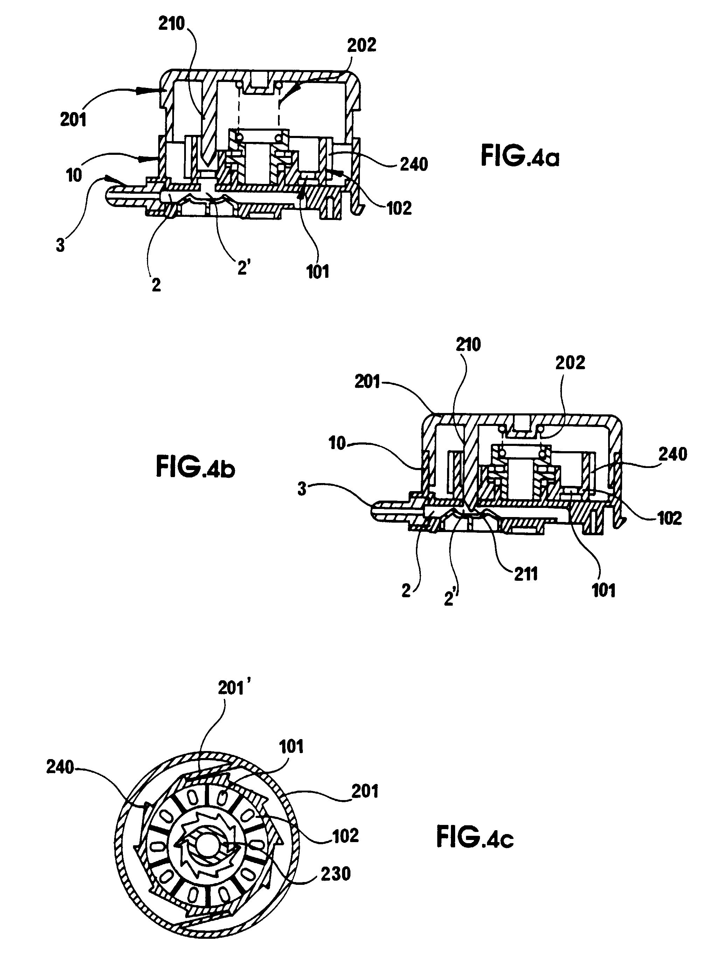

En référence à la figure 1, il est représenté schématiquement un appareil d'inhalation selon un mode de réalisation particulier de l'invention. Cet appareil 1 comprend une source d'écoulement d'air 300, représentée plus en détail sur la figure 2, ainsi qu'un dispositif de pré-dosage 100 et un dispositif de transfert 200, dont deux modes de réalisation différents sont représentés respectivement sur les figures 3a à 3d et 4a à 4c.

L'inhalateur 1 comporte un canal d'expulsion 2 qui, d'un côté s'étend en direction d'un orifice de sortie 3, avantageusement disposé dans un embout buccal 8, et de l'autre côté, se connecte à la source d'écoulement d'air 300.

La source d'écoulement d'air 300 comporte une pompe 301, de préférence précontrainte déclenchée par l'inhalation de l'utilisateur. Un exemple d'une telle source d'écoulement d'air est divulgué dans le document WO 93/18812, et son fonctionnement ainsi que sa mise en oeuvre ne seront par conséquent que brièvement développés en référence à la figure 2, étant entendu que toutes les variantes divulguées dans le document WO 93/18812 sont également applicables ici. De même, il est clair que d'autres dispositifs de source d'écoulement d'air connus pourraient être utilisés ici, du moment qu'ils comportent une pompe permettant de créer un écoulement d'air dans le canal d'expulsion de l'inhalateur, ledit écoulement d'air étant déclenché lors de l'inhalation de l'utilisateur.

En se référant à la figure 2, un exemple d'une telle source d'écoulement d'air 300 est donc représentée schématiquement. Cette source d'écoulement d'air 300 comprend une pompe 301 qui est précontrainte manuellement. Cette pompe 301 comporte un piston 302 qui coulisse dans une chambre 303 entre une position de repos et une position d'actionnement, ladite chambre 303 étant reliée au canal d'expulsion 2 de l'inhalateur, par exemple par l'intermédiaire d'un clapet de sortie 315 de la chambre 303. Le piston 302 est sollicité vers sa position d'actionnement par un ressort 304 qui peut être comprimé manuellement au moyen d'un organe de compression 309 solidaire du fond 311 de l'inhalateur. Pendant l'étape de compression du ressort 304, le piston 302 est maintenu dans sa position de repos par un organe de blocage 305, en l'occurrence un anneau fendu 305 logé dans le corps 307 formant la chambre 303 et maintenu dans une rainure 306 du piston au moyen d'un manchon coulissant 308 qui empêche ledit anneau fendu 305 de sortir de ladite rainure 306 du piston 302. Ainsi l'utilisateur arme la pompe en comprimant le ressort 304 au moyen de l'organe de compression 309 en appuyant sur le fond 311 de l'inhalateur. La source d'écoulement d'air, pour être coordonnée avec l'inhalation du patient, comporte un élément de type levier 320 qui, à l'état de repos, recouvre et ferme une ouverture 330 ménagée dans le corps de l'inhalateur, au moyen de son extrémité 322. Ledit levier 320 peut pivoter autour de son point d'attache 321, ledit point d'attache étant situé au voisinage d'un élément 323 solidaire dudit manchon coulissant 308 mentionné précédemment. Ainsi, lorsque l'utilisateur inhale par l'embout buccal 8, il crée une dépression dans l'espace 340 situé en face de l'ouverture 330 du corps de l'inhalateur ce qui provoque le basculement vers l'intérieur du levier 320 au niveau de son point d'encrage 321, celui-ci agissant sur l'élément 323 solidaire du manchon 308 en déplaçant ce dernier légèrement vers le haut. Un déplacement du manchon 308 vers le haut libère l'anneau fendu 305 qui sous l'effet de la force exercée par le ressort 304 sur ledit piston 302 est forcé vers l'extérieur hors de la rainure 306 du piston 302, de sorte que le piston 302 n'est plus retenu par l'organe de blocage et peut, sous l'effet de la force du ressort 304 coulisser dans la chambre 303. Le piston 302 chasse ainsi l'air contenue dans la chambre 303 via le clapet de sortie 315 de cette chambre dans le canal d'expulsion 2 pour expulser la dose de produit, cette expulsion étant déclenchée par l'inhalation de l'utilisateur.

En référence aux figures 3a à 3d, et 4a à 'd, sont représentés deux modes de réalisation différent du dispositif de pré-dosage et du dispositif de transfert pour l'appareil d'inhalation de l'invention.

Pour éviter une contamination et/ou une humidification de l'ensemble de la poudre lorsqu'une contamination et/ou une humidification d'une partie de la poudre se produit, pour garantir une reproductibilité parfaite de la dose de poudre et pour éviter toute perte de produit dans un éventuel volume mort d'un réservoir unique, l'invention prévoit d'utiliser un dispositif de pré-dosage en réservoirs individuels. Dans l'exemple représenté sur les figures 3a à 3d, le dispositif de pré-dosage 100 comporte une pluralité de réservoirs individuels 101, en l'occurrence dix. Ces réservoirs individuels 101 sont ménagés dans un support, en l'occurrence un disque rigide 102 qui est mobile en rotation dans le corps supérieur 10 de l'inhalateur 1, dans lequel est formé le canal d'expulsion 2. Chaque réservoir 101 comporte un orifice d'entrée et un orifice de sortie adapté à s'aligner sur ledit canal d'expulsion 2, de sorte que lorsqu'un réservoir 101 est disposé dans ledit canal d'expulsion 2, la poudre contenue dans celui-ci peut être expulsée par l'écoulement d'air crée par la source d'écoulement d'air 300 décrite précédemment. Avantageusement, le disque 102 contenant les réservoirs 101 tourne dans le corps 10 de l'inhalateur de manière étanche et des joints (non représentés) peuvent être prévus autour des jonctions entre les orifices d'entrée et de sortie du réservoir et le canal d'expulsion, lorsque ledit réservoir est disposé dans ledit canal d'expulsion. Ces joints peuvent servir à garantir une étanchéité lors de l'expulsion de la dose par l'écoulement d'air.

Le dispositif de transfert 200 prévu pour déplacer l'un des réservoirs 101 dans ledit canal d'expulsion 2 avant chaque utilisation de l'inhalateur, comporte un poussoir 201 déplaçable axialement sur le corps 10 de l'inhalateur entre une position de repos et une position d'actionnement. Ledit poussoir est sollicité vers sa position de repos par un ressort 202. Comme représenté sur les figures 3a, 3b, 3c et 3d, le dispositif de transfert 200 comporte en outre un doigt 210 dont l'extrémité 211 est souple et peut s'incurver lorsque le poussoir 201 est déplacé de sa position de repos (figure 3a) vers sa position d'actionnement (figure 3b). L'extrémité 211 dudit doigt 210 coopère avec un logement 101' correspondant à chaque réservoir individuel 101 du dispositif de pré-dosage. Comme représenté sur la figure 3c, l'extrémité 211 du doigt 210 butte contre une paroi 101" dudit logement 101' solidaire de chaque réservoir 101, et une continuation du déplacement axial du poussoir 201 vers sa position d'actionnement représentée sur la figure 3b, entraîne une rotation dudit dispositif de pré-dosage 100 et amène ainsi le prochain réservoir 101 dans le canal d'expulsion 2 de l'inhalateur 1. Pour éviter une rotation en sens inverse, un cliquet anti-retour 230 peut avantageusement être disposé à l'intérieur du disque rigide 102 du dispositif de pré-dosage. Ledit cliquet anti-retour 230 pourrait par exemple coopérer avec une denture circonférentielle dudit disque, comme représenté sur la figure 3c. Lorsque le poussoir 201 est dans sa position d'actionnement représentée sur la figure 3b, un réservoir 101 est placé dans le canal d'expulsion 2 de l'inhalateur, et le ressort 202 ramène ensuite le poussoir 201 vers sa position de repos représentée sur la figure 3a. L'utilisateur n'a alors plus qu'à armer la source d'écoulement d'air et à inhaler, ce qui déclenche ledit écoulement d'air qui vient chasser la dose de produit contenu dans ledit réservoir 101 et l'expulse à travers l'orifice 3 vers l'utilisateur.

En référence aux figures 4a, 4b et 4c, un autre mode de réalisation du dispositif de pré-dosage et du dispositif de transfert est représenté. Dans cet exemple, le dispositif de pré-dosage est disposé au dessus du canal d'expulsion 2 et une chambre de dosage 2' peut être ménagée dans ledit canal 2 pour recueillir une dose de produit en vue de son expulsion. Ainsi, le dispositif de pré-dosage 100 comporte également un disque rigide en tant que support 102, ledit disque comportant une pluralité de réservoirs 101 réalisée sous la forme d'ouverture traversante à travers ledit disque 102, et disposée circonférentiellement sur celui-ci. Chaque réservoir 101, c'est à dire chaque ouverture, est obturée des deux côtés par une couche imperméable de sorte que chaque réservoir 101 est fermé hermétiquement par rapport à l'atmosphère et les doses de produit contenues dans les réservoirs 101 sont empêchées d'être contaminées et/ou humidifiées. Le dispositif de transfert 200 comporte également un poussoir 201 qui, contrairement au poussoir du premier mode de réalisation décrit ci-dessus, est mobile à la fois axialement et en rotation par au corps de l'inhalateur. Ainsi, pour disposer un réservoir 101 en regard de la chambre de dosage 2' disposée dans le canal d'expulsion 2 de l'inhalateur, le poussoir 201 est tourné, par exemple dans le sens alors des aiguilles d'une montre (figure 4c). Ce poussoir 201 comporte une ou plusieurs languettes 201' coopérant avantageusement avec une denture 240 solidaire du support 102 desdits réservoir 101, de sorte que cette rotation fait tourner ledit support et déplace un réservoir 101 en regard de ladite chambre de dosage 2' de l'inhalateur. Le poussoir 201 est ensuite tourné en sens inverse pour être ramené vers sa position initiale. Pendant cette opération, on prévoit avantageusement un cliquet anti-retour 230 solidaire du corps 10 de l'inhalateur et qui coopère par exemple avec une denture interne solidaire du disque 102, pour éviter que celui-ci ne se déplace lorsque le poussoir 201 est ramené dans sa position initiale. Le poussoir 201 comporte en outre un doigt 210 qui est disposé en regard de la chambre de dosage 2' et d'un réservoir 101, lorsque le poussoir 201 est dans sa position initiale. Ce doigt 210 est avantageusement muni d'une extrémité 211 adaptée à percer les couches de fermeture de chaque réservoir 101 de sorte que lors d'une pression sur ledit poussoir 201 celui-ci se déplace axialement de sa position représentée sur la figure 4a vers sa position représentée sur la figure 4b, l'extrémité 211 du doigt 210 transperçant les deux parois de fermeture et transférant la dose de produit dans la chambre de dosage 2' dans le canal d'expulsion 2 de l'inhalateur. Ainsi une inhalation déclenche la source d'écoulement d'air décrite précédemment 300 et envoie un écoulement d'air à travers le canal d'expulsion 2 qui vide la chambre de dosage 2' de sa dose de poudre. Le ressort 202 ramène ensuite le poussoir 201 vers sa position de repos de la figure 4a.

Bien entendu, la description ci-dessus des différents dispositifs formant la combinaison de l'invention, à savoir le dispositif de pré-dosage, le dispositif de transfert, et la source d'écoulement d'air peuvent varier d'une manière quelconque connue et les exemples décrits ne sont pas limitatifs. Le point essentiel de l'invention réside dans le fait que chacun desdits dispositifs fonctionne de manière optimale pour fournir un inhalateur déclenché par l'inhalation qui soit le plus efficace possible. Ainsi, un dispositif de pré-dosage comportant une bande souple en tant que support plutôt qu'un disque rigide est applicable à la présente invention. Dans ce cas, un dispositif de transfert correspondant permettra d'entraîner la bande et d'ouvrir les réservoirs qu'elle supporte pour transférer une dose de produit dans le canal d'expulsion de l'inhalateur avant chaque inhalation.

## Revendications

1. Appareil d'inhalation (1) destiné à distribuer des doses précises et reproductibles de produit pulvérulent, ledit appareil comportant un canal d'expulsion (2) débouchant dans un orifice de sortie (3), et comprend en combinaison :
- un dispositif de transfert (200) pour transférer en totalité une dose de produit dans ledit canal d'expulsion (2), lors de chaque actionnement de l'appareil d'inhalation,
- une source d'écoulement d'air (300) comprenant une pompe (301) déclenchable par l'inhalation de l'utilisateur, ladite source d'écoulement d'air (300) envoyant un écoulement d'air comprimé dans ledit canal d'expulsion, (2), en direction dudit orifice de sortie (3), pour expulser ladite dose de produit, caractérisé en ce qu'il comprend :
- des réservoirs individuels (101) pré-dosés, chaque réservoir (101) contenant une dose de produit et étant fermé hermétiquement par rapport à l'atmosphère, et ledit dispositif de transfert (200) comportant des moyens d'ouverture et de transfert pour ouvrir un réservoir (101) et transférer la dose de produit dans le canal d'expulsion (2) avant une inhalation de l'utilisateur.

2. Appareil d'inhalation selon la revendication 1, dans lequel ladite pompe (301) est précontrainte et comporte un piston (302) coulissant dans une chambre (303) reliée audit canal d'expulsion (2), ledit piston (302) étant sollicité par un ressort(304), ledit ressort (304) étant comprimable manuellement et ledit piston (302) étant retenu contre la force exercée par ledit ressort (304) comprimé par un organe de blocage (305) , ledit organe de blocage (305) étant libéré par l'inhalation de l'utilisateur, de sorte que le ressort (304) se détend en déplaçant ledit piston (302) dans ladite chambre (303), créant ainsi un écoulement d'air dans ledit canal d'expulsion (2).

3. Appareil d'inhalation selon les revendications 1 ou 2, dans lequel ledit dispositif de pré-dosage (100) comporte un support (102) pourvu d'au moins un réservoir (101), chaque réservoir (101) comportant une dose précise de produit, le dispositif de transfert (200) comportant des moyens d'entraînement dudit support (102) pour amener un desdits réservoirs (101) dans le canal d'expulsion (2) avant une inhalation de l'utilisateur.

## Patentansprüche

1. Inhalationsgerät (1), das dazu dient, genaue und reproduzierbare Dosissen eines Pulvers abzugeben, wobei das Gerät einen Ausstoßkanal (2) umfaßt, der in einer Ausgangsöffnung (3) mündet, und das Gerät weiterhin in Kombination folgende Bestandteile umfaßt:
- eine Überführungseinrichtung (200), die dazu dient, bei jeder Betätigung des Inhalationsgerätes eine Dosis des Produkts vollständig in den Ausstoßkanal (2) zu überführen,
- eine Quelle (300) für einen Luftstrom, die eine durch die Inhalation des Verwenders auslösbare Pumpe (301) umfaßt, wobei die Quelle (300) für den Luftstrom einen Strom komprimierter Luft in den Ausstoßkanal (2) in Richtung der Austrittsöffnung (3) aussendet, um die Dosis des Produktes auszustoßen,
dadurch gekennzeichnet, daß das Gerät weiterhin folgende Bestandteile umfaßt
- einzelne vordosierte Behälter (101), wobei jeder Behälter (101) eine Dosis des Produkts enthält und bezüglich der Atmosphäre hermetisch verschlossen ist, und wobei die Übertragungseinrichtung (200) Einrichtungen zum Öffnen und Überführen aufweist, die dazu dienen, vor einer Inhalation des Verwenders einen Behälter (101) zu öffnen und die Dosis des Produktes in den Ausstoßkanal (2) zu überführen.

2. Inhalationsgerät nach Anspruch 1, bei dem die Pumpe (301) vorgespannt ist und einen Kolben (302) umfaßt, der in einer Kammer (303) gleitet, die mit dem Ausstoßkanal (2) verbunden ist, wobei der Kolben (302) durch eine Feder (304) vorgespannt ist, wobei die Feder (304) manuell zusammendrückbar ist und der Kolben (302) gegen die von dieser zusammengedrückten Feder (304) ausgeübte Kraft durch ein Blockierorgan (305) zurückgehalten wird, wobei das Blockierorgan (305) durch die Inhalation des Verwenders so freigegeben wird, daß sich die Feder (304) entspannt und dabei den Kolben (302) in der Kammer (303) verschiebt, wodurch im Ausstoßkanal (2) ein Luftstrom erzeugt wird.

3. Inhalationsgerät nach den Ansprüchen 1 oder 2, bei dem die Vorrichtung (100) zum Vordosieren einen Träger (102) umfaßt, der mit wenigstens einem Behälter (101) versehen ist, wobei jeder Behälter (101) eine genaue Dosis des Produkts enthält, und wobei die Übertragungsvorrichtung (200) Antriebseinrichtungen für den Träger (102) umfaßt, um einen der Behälter (101) vor einer Inhalation durch den Verwender in den Ausstoßkanal (2) zu bewegen.

## Claims

1. An inhaler (1) for dispensing accurate and reproducible doses of powder, said inhaler including an expulsion channel (2) opening out in an outlet orifice (3), and comprising in combination:
- a transfer device (200) for transferring a dose of powder in full into said expulsion channel (2) on each actuation of the inhaler; and
- an air flow source (300) comprising a pump (301) triggerable by the user inhaling, said air flow source (300) sending a flow of compressed air into said expulsion channel (2) towards said outlet orifice (3) to expel said dose of powder;
caracterized in that the inhaler comprises:
- individual pre-dosed reservoirs (101), each reservoir (101) containing one dose of powder, and being hermetically sealed from the atmosphere; and
- said transfer device (200) including opening and transfer means for opening a reservoir (101) and transferring its dose of powder into the expulsion channel (2) before the user inhales.

2. An inhaler according to claim 1, in which said pump (301) is prestressed and includes a piston (302) sliding in a chamber (303) connected to said expulsion channel (2), said piston (302) being biased by a spring (304), said spring (304) being manually compressible, and said piston (302) being held against the force exerted by said compressed spring (304) by a locking member (305), said locking member (305) being released by the user inhaling so that the spring (304) relaxes and thereby displaces said piston (302) in said chamber (303), thereby establishing a flow of air through said expulsion channel (2).

3. An inhaler according to claim 1 or 2, in which said pre-dosing device (100) comprises a support (102) provided with at least one reservoir (101), each reservoir (101) containing an accurate dose of powder, the transfer device (200) including means for driving said support (102) to bring one of said reservoirs (101) into the expulsion channel (2) prior to the user inhaling.
